# EUROPEAN PATENT APPLICATION

(11) **EP 4 212 619 A1**
(43) Date of publication of application: **19.07.2023**
(21) Application number: 21865931.6
(22) Date of filing: 03.09.2021
(51) Int. Cl.: C12N 5/10, C12N 9/22, C12N 15/87, C12N 15/85, C07K 14/705, C12N 15/90, C12N 15/113, A61P 35/00

(54) **METHOD FOR PERFORMING GENE EDITING ON TARGET SITE IN CELL**

(30) Priority: 10.09.2020 CN 202010949701
(71) Applicant: BRL Medicine Inc., Shanghai 201109 (CN)
(72) Inventor: ZHANG, Jiqin, Shanghai 201109 (CN); DU, Bing, Shanghai 201109 (CN); LIU, Mingyao, Shanghai 201109 (CN); XI, Zaixi, Shanghai 201109 (CN)
(74) Representative: Haseltine Lake Kempner LLP
(86) International application number: PCT/CN2021/116531
(87) International publication number: WO 2022/052877

(57) **Abstract**

A method for performing gene editing on a target site in a cell, specifically a method for performing gene editing on a target site of a cell genome, comprising: (a) providing a cell to be genetically edited; (b) introducing into the cell (i) a gene editing enzyme or the encoding nucleic acid thereof or a first expression vector expressing the gene editing enzyme; and (ii) gRNA or a second expression vector expressing the gRNA, and performing gene editing on a target site of the cell genome, the gRNA directing the gene editing enzyme to perform fixed-point cutting on the target site; the targeting sequence of the gRNA targeting comprises one or more of the sequences shown in SEQ ID. No. 1-9. The method enables efficient gene editing to be performed on the target site.

## Description

### TECHNICAL FIELD

The present disclosure relates to the field of biotechnology, in particular to a method for performing gene editing on a target site in a cell.

### BACKGROUND

CRISPR/Cas9 system is an acquired immune mechanism derived from archaea and bacteria that protects against the invasion of exogenous DNA fragments such as plasmids and phages. The system mainly functions by CRISPR sequences and loci encoding Cas proteins. Cas9 nucleases, belong to the type II family, are currently most commonly used, in which only a single effector protein can function. When exogenous DNAinvades bacteria, the type II CRISPR system first integrates the invading DNA between CRISPR repeat sequences. Subsequently, CRISPR RNAs (crRNAs) containing invading DNA sequences are transcribed and processed. Thereafter, trans-activating crRNA (tracrRNA) binds to crRNA and eventually forms a complex with the Cas9 protein. Finally, the Cas9 protein acts as an endonuclease to trigger DNA double-strand breaks through its HNH and RuvC-like domains. Double-strand DNA breaks trigger damage repair mechanisms, and when homologous templates are present, cells can undergo precise homologous recombination repair, which allows the insertion or replacement of exogenous sequences. So far, CRISPR/Cas9 technology has been successfully applied in many fields and shows broad prospects. However, there are still many difficulties in precise gene therapy using homologous recombination, and one of the main limiting factors is that the efficiency of homologous recombination is very low. Although a number of studies have reported a variety of methods to increase homologous recombination efficiency, increasing evidence shows that these methods are not applicable for all types of cells and the effects are not consistent. Therefore, finding personalized approaches to effectively improve homologous recombination in specific cells is particularly important for cell type-based cell therapy.

Chimeric Antigen Receptor T-Cell (CAR-T) is a novel adoptive immunotherapy emerging in recent years. It genetically modifies patients' T cells *in vitro* and reinfuses them into patients after expansion, achieving targeted killing of tumors. At present, three CAR-T products have been approved by the US Food and Drug Administration for the clinical treatment of hematological malignancies, demonstrating the great success of CAR-T technology. However, as emerging technologies, CAR-T technology still faces many challenges in various aspects and has huge room for improvement. First, the most commonly used method nowadays is to introduce exogenous sequences using viral systems. However, the use of virus systems has the problems of high preparation cost and potential safety risks with random insertion. Secondly, traditional technologies, including viral preparation are unable to achieve precise insertion of exogenous sequences into the genome, which can cause poor cell homogeneity and affect the therapeutic effect, and fail to engineer T cells diversely. In addition, CAR-T therapy is currently a personalized treatment, which has problems such as high production cost, long preparation time, limited effects and other problems. Therefore, it is important to achieve site-specific integration of artificial modified elements such as CAR in T cells using technologies such as CRISPR/Cas9 to promote the development of existing T cell therapy. On the one hand, it can avoid the uncertainty caused by random insertion of exogenous sequences and improve the homogeneity and robustness of cell products. On the other hand, it can achieve versatile transformation of T cells, which can not only construct universal CAR-T cells in one step, construct enhanced cell products by regulating endogenous genes, but also achieve the dynamic expression of artificial elements, which is of great help to expand the application of current technologies. However, due to the property of T cells, the efficiency of site-directed integration of exogenous sequences by homologous recombination is very low. Therefore, there is an urgent need to develop a method to improve the efficiency of homologous recombination in T cells in this field.

### CONTENT OF THE PRESENT INVENTION

One of the purposes of the present invention is to provide a novel method to improve the efficiency of homologous recombination of an exogenous sequence in a cell (especially T cell).

The invention provides a method of gene editing for genes AAVS1, PD 1, TRAC and B2M, respectively.

The first aspect of the present invention provides a method for performing gene editing on a target site of a cellular genome comprising:
(a) providing a cell to be gene-edited;
(b) introducing (i) an enzyme for gene editing, or a nucleic acid encoding the same, or a first expression vector expressing the enzyme for gene editing; and (ii) gRNA, or a second expression vector expressing the gRNA into the cell, and performing gene editing on a target site of the cellular genome, wherein the gRNA guides the enzyme for gene editing to perform site-directed cleavage on the target site;
wherein, the targeting sequence targeted by the gRNA comprises one or more of the sequences shown in SEQ ID NO: 1-9.

In another preferred embodiment, the gene editing comprises gene knock-out and gene-targeted integration.

In another preferred embodiment, when the targeting sequence targeted by the gRNA comprises one or more of the sequences shown in SEQ ID NO: 1-6, the gene editing is gene-targeted integration.

In another preferred embodiment, when the targeting sequence targeted by the gRNA comprises one or more of the sequences shown in SEQ ID NO: 7-9, the gene editing is gene knock-out.

In another preferred embodiment, the enzyme for gene editing is selected from the group consisting of CRISPR related protein (Cas) polypeptide, TALEN enzyme, ZFN enzyme, or combinations thereof.

In another preferred embodiment, the enzyme for gene editing is derived from a microorganism; preferably derived from a bacterium.

In another preferred embodiment, the source of the enzyme for gene editing is selected from the group consisting of *Streptococcus pyogenes*, *Staphylococcus aureus*, *Streptococcus canis* or combinations thereof.

In another preferred embodiment, the enzyme for gene editing comprises a wild-type or mutated enzyme for gene editing.

In another preferred embodiment, the enzyme for gene editing is selected from the group consisting of Cas9, Cas12, Cas13, Cms1, MAD7, Cas3, Cas8a, Cas8b, Cas10d, Cse1, Csy1, Csn2, Cas4, Cas10, Csm2, Cmr5, Fok1, Cpf1 or combinations thereof.

In another preferred embodiment, the gRNA comprises crRNA, tracrRNA, sgRNA.

In another preferred embodiment, the gRNA targets and binds to the target site of the genome.

In another preferred embodiment, the gRNA comprises a gRNA without or with a modification.

In another preferred embodiment, the gRNA with a modication comprises a chemical modification on a base.

In another preferred embodiment, the chemical modification comprises methylation modification, methoxy modification, fluorination modification or sulfur modification.

In another preferred embodiment, the gene editing comprises *in vivo* gene editing, *in vitro* gene editing.

In another preferred embodiment, the gene editing comprises CRISPR based gene editing.

In another preferred embodiment, the target site is selected from the group consisting of AAVS1, PD1, TRAC, B2M or combinations thereof.

In another preferred embodiment, the target sequence targeted by the gRNA for AAVS1 comprises the sequence shown in SEQ ID NO: 3.

In another preferred embodiment, the target sequence targeted by the gRNA for PD1 comprises the sequence shown in SEQ ID NO: 1 and/or SEQ ID NO: 2.

In another preferred embodiment, the target sequence targeted by the gRNA for TRAC comprises the sequence shown in SEQ ID NO: 4 and/or SEQ ID NO: 5.

In another preferred embodiment, the target sequence targeted by the gRNA for B2M comprises the sequence shown in SEQ ID NO: 6.

In another preferred embodiment, the gene editing comprises a site-directed knock-in of a DNA donor.

In another preferred embodiment, the DNA donor is double-stranded DNA.

In another preferred embodiment, the DNA donor comprises a first homology arm and a second homology arm, wherein the first homology arm and the second homology arm are capable of initiating a cell-mediated homologous recombination of the DNA donor at the target site of the cellular genome, and the sequence lengths of each of the first homology arm and the second homology arm are independently 200-2000 bp, preferably 400-1000 bp, more preferably 700-900 bp.

In another preferred embodiment, the first homology arm is homologous to the sequence upstream-side (or left-side) of the cleavage site of the target site in the genome, while the second homology arm is homologous to the sequence downstream-side (or right-side) of the cleavage site of the target site in the genome.

In another preferred embodiment, the DNA donor comprises a target gene.

In another preferred embodiment, the target gene comprises a coding sequence of a chimeric antigen receptor or a TCR.

In another preferred embodiment, the sequence length of the DNA donor is 50 bp-5000 bp, preferably 80 bp-4000 bp.

In another preferred embodiment, the sequence length of the target gene is 50 bp-3000 bp, preferably 1000 bp-2000 bp.

In another preferred embodiment, the sequence length of the coding sequence of the chimeric antigen receptor or TCR is 50 bp-3000 bp, preferably 1000 bp-2000 bp.

In another preferred embodiment, the ratio (D1/D2) of the sequence length of the first homology arm (D1) to the sequence length of the second homology arm (D2) is (0.8-1.2) : (0.5-1.5), preferably (0.9-1.1) : (0.7-1.3), more preferably, 1:1.

In another preferred embodiment, the chimeric antigen receptor contains an antigen binding domain targeting a tumor cell marker.

In another preferred embodiment, the chimeric antigen receptor comprises an antigen binding domain targeting a tumor cell marker, an optional hinge region, a transmembrane domain and an intracellular signal transduction binding domain.

In another preferred embodiment, the tumor cell marker is selected from the group consisting of folate receptor α, 5T4, αvβ6 integrin, BCMA, B7-H3, B7-H6, CAIX, CD16, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRα, GD2, GD3, phosphatidylinositol proteoglycan-3 (GPC3), HLA-A1+MAGE1, HLA-A2+MAGE1, HLA-A3+MAGE1, HLA-A1+NY-ESO-1, HLA-A2+NY-ESO-1, HLA-A3+NY-ESO-1, IL-11Rα, IL-13Rα2, Lambda, Lewis-Y, Kappa, mesothelin, Muc1, Muc16, NCAM, NKG2D ligand, NY-ESO-1, PRAME, PSCA, PSMA, ROR1, SSX, survivin, TAG72, TEM, VEGFR2, WT-1, or combinations thereof.

In another preferred embodiment, the hinge region is a hinge region of a protein selected from the group consisting of CD8, Ig (immunoglobulin) hinge, CD28, or combinations thereof.

In another preferred embodiment, the transmembrane domain is a transmembrane domain of a protein selected from the group consisting of CD8α, CD8β, CD28, CD33, CD37, CD5, CD16, ICOS, CD9, CD22, CD134, CD137, CD154, CD19, CD45, CD4, CD3ε, CD3γ, CD3ζ, or combinations thereof.

In another preferred embodiment, the intracellular signal transduction binding domain comprises a costimulatory signaling molecule and/or a primary signal transduction domain.

In another preferred embodiment, the costimulatory signaling molecule is selected from the group consisting of OX40, CD28, CD30, CD40, CD70, CD134, 4-1BB (CD137), LIGHT, DAP10, CDS, ICAM-1, CD278 (ICOS), TLR1, TLR2, TLR3, TLR4, TLR5, TLR6, TLR7, TLR8, TLR9, TLR10, CARD11, CD2, CD7, CD27, CD54 (ICAM), CD83, LAT, NKD2C, SLP76, TRIM, ZAP70, or combinations thereof.

In another preferred embodiment, the primary signal transduction domain is selected from the group consisting of FcRy, FcRβ, CD3γ, CD3δ, CD3ε, CD3ζ, CD22, CD79a, CD79b, CD66d, or combinations thereof.

In another preferred embodiment, the sequence of the DNA donor is shown in any one of SEQ ID NO: 12-38.

In another preferred embodiment, the cell comprises a primary cell and a subcultured cell.

In another preferred embodiment, the cell comprises a T cell.

In another preferred embodiment, the cell comprises a CD3+ T cell, a CD4+ helper T cell, a CD4+ regulatory T cell, a CD8+ T cell and a memory T cell.

In another preferred embodiment, the vector comprises a viral vector.

In another preferred embodiment, the viral vector is selected from the group consisting of an adeno-associated viral vector, a lentiviral vector, or combinations thereof.

In another preferred embodiment, the method for introducing comprises electrotransport, vector transformation, transfection, heat shock, electroporation, transduction, gene gun, microinjection, liposome transfection and lentivirus infection.

In another preferred embodiment, the method is an *in vitro* method.

In another preferred embodiment, the method is non-diagnostic or non-therapeutic.

The second aspect of the present invention provides a gRNA for gene editing on a target site of a cellular genome, wherein the targeting sequence targeted by the gRNA comprises one or more of the sequences shown in SEQ ID NO: 1-9.

In another preferred embodiment, the targeting sequence targeted by the gRNA comprises one or more of the sequences shown in SEQ ID NO: 1-6.

In another preferred embodiment, the targeting sequence targeted by the gRNA comprises one or more of the sequences shown in SEQ ID NO: 7-9.

In another preferred embodiment, the gRNA comprises a gRNA without or with a modification.

In another preferred embodiment, the gRNA with a modification comprises a chemical modification on a base.

In another preferred embodiment, the chemical modification comprises methylation modification, methoxy modification, fluorination modification or sulfur modification.

The third aspect of the present invention provides a reaction system for gene editing on a target site of a cellular genome comprising:
(a) a DNA donor, wherein the DNA donor is double-stranded DNA;
(b) an enzyme for gene editing or nucleic acid encoding the same, or a first expression vector expressing the enzyme for gene editing;
(c) a gRNA or a second expression vector expressing the gRNA;
wherein, the targeting sequence targeted by the gRNA comprises one or more of the sequences shown in SEQ ID NO: 1-9.

In another preferred embodiment, the gene editing comprises gene knock-out and gene-targeted integration.

In another preferred embodiment, when the targeting sequence targeted by the gRNA comprises one or more of the sequences shown in SEQ ID NO: 1-6, the gene editing is gene-targeted integration.

In another preferred embodiment, when the targeting sequence targeted by the gRNA comprises one or more of the sequences shown in SEQ ID NO: 7-9, the gene editing is gene knock-out.

In another preferred embodiment, the target site is selected from the group consisting of AAVS1, PD1, TRAC, B2M or combinations thereof.

In another preferred embodiment, the target sequence targeted by the gRNA for AAVS1 comprises the sequence shown in SEQ ID NO: 3.

In another preferred embodiment, the target sequence targeted by the gRNA for PD1 comprises the sequence shown in SEQ ID NO: 1 and/or SEQ ID NO: 2.

In another preferred embodiment, the target sequence targeted by the gRNA for TRAC comprises the sequence shown in SEQ ID NO: 4 and/or SEQ ID NO: 5.

In another preferred embodiment, the target sequence targeted by the gRNA for B2M comprises the sequence shown in SEQ ID NO: 6.

In another preferred embodiment, the DNA donor comprises a first homology arm and a second homology arm, wherein the first homology arm and the second homology arm are capable of initiating an immune cell-mediated homologous recombination of the DNA donor at the target site of the genome of the immune cell, and the sequence lengths of each of the first homology arm and the second homology arm are independently 200-2000 bp, preferably 400-1000 bp, more preferably 700-900 bp.

In another preferred embodiment, the DNA donor comprises a target gene.

In another preferred embodiment, the target gene comprises a coding sequence of a chimeric antigen receptor or a TCR.

In another preferred embodiment, the chimeric antigen receptor contains an antigen binding domain targeting a tumor cell marker.

In another preferred embodiment, the chimeric antigen receptor comprises an antigen binding domain targeting a tumor cell marker, an optional hinge region, a transmembrane domain and an intracellular signal transduction binding domain.

In another preferred embodiment, the tumor cell marker is selected from the group consisting of folate receptor α, 5T4, αvβ6 integrin, BCMA, B7-H3, B7-H6, CAIX, CD16, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRα, GD2, GD3, phosphatidylinositol proteoglycan-3 (GPC3), HLA-A1+MAGE1, HLA-A2+MAGE1, HLA-A3+MAGE1, HLA-A1+NY-ESO-1, HLA-A2+NY-ESO-1, HLA-A3+NY-ESO-1, IL-11Rα, IL-13Rα2, Lambda, Lewis-Y, Kappa, mesothelin, Muc1, Muc16, NCAM, NKG2D ligand, NY-ESO-1, PRAME, PSCA, PSMA, ROR1, SSX, survivin, TAG72, TEM, VEGFR2, WT-1, or combinations thereof.

In another preferred embodiment, the hinge region is a hinge region of a protein selected from the group consisting of CD8, Ig (immunoglobulin) hinge, CD28, or combinations thereof.

In another preferred embodiment, the transmembrane domain is a transmembrane domain of a protein selected from the group consisting of CD8α, CD8β, CD28, CD33, CD37, CD5, CD16, ICOS, CD9, CD22, CD134, CD137, CD154, CD19, CD45, CD4, CD3ε, CD3γ, CD3ζ, or combinations thereof.

In another preferred embodiment, the intracellular signal transduction binding domain comprises a costimulatory signaling molecule and/or a primary signal transduction domain.

In another preferred embodiment, the costimulatory signaling molecule is selected from the group consisting of OX40, CD28, CD30, CD40, CD70, CD134, 4-1BB (CD137), LIGHT, DAP10, CDS, ICAM-1, CD278 (ICOS), TLR1, TLR2, TLR3, TLR4, TLR5, TLR6, TLR7, TLR8, TLR9, TLR10, CARD11, CD2, CD7, CD27, CD54 (ICAM), CD83, LAT, NKD2C, SLP76, TRIM, ZAP70, or combinations thereof.

In another preferred embodiment, the primary signal transduction domain is selected from the group consisting of FcRy, FcRβ, CD3γ, CD3δ, CD3ε, CD3ζ, CD22, CD79a, CD79b, CD66d, or combinations thereof.

In another preferred embodiment, the cell comprises a primary cell and a subcultured cell.

In another preferred embodiment, the cell comprises a T cell.

In another preferred embodiment, the cell comprises a CD3+ T cell, a CD4+ helper T cell, a CD4+ regulatory T cell, a CD8+ T cell and a memory T cells.

In another preferred embodiment, the first homology arm is homologous to the sequence upstream-side (or left-side) of the cleavage site of the target site in the genome, while the second homology arm is homologous to the sequence downstream-side (or right-side) of the cleavage site of the target site in the genome.

In another preferred embodiment, the sequence length of the DNA donor is 50 bp-5000 bp, preferably 80 bp-4000 bp.

In another preferred embodiment, the sequence length of the target gene is 50 bp-3000 bp, preferably 1000 bp-2000 bp.

In another preferred embodiment, the sequence length of the coding sequence of the chimeric antigen receptor or TCR is 50 bp-3000 bp, preferably 1000 bp-2000 bp.

In another preferred embodiment, the sequence of the DNA donor is shown in any one of SEQ ID NO: 12-38.

In another preferred embodiment, the vector comprises a viral vector.

In another preferred embodiment, the viral vector is selected from the group consisting of an adeno-associated viral vector, a lentiviral vector, or combinations thereof.

The fourth aspect of the present invention provides a kit for gene editing comprising:
i) a first container and a DNA donor contained therein, wherein the DNA donor is double-stranded DNA;
ii) a second container and an enzyme for gene editing, or a nucleic acid encoding thereof or a first expression vector expressing the enzyme for gene editing contained therein; and
iii) a third container and a gRNA or a second expression vector expressing the gRNA contained therein, and the targeting sequence targeted by the gRNA comprises one or more of the sequences shown in SEQ ID NO: 1-9.

In another preferred embodiment, the first container, the second container and the third container are the same container or different containers.

The fifth aspect of the present invention provides a gene-edited cell prepared by the method of the first aspect of the present invention.

The sixth aspect of the present invention provides a use of the cell of the fifth aspect of the present invention in the preparation of a product for tumor immunotherapy or cancer immunotherapy.

Those of skill in the art should be understood that, within the scope of the present invention, each of the above technical features and the technical features specifically described in the following (such as the examples) in the present invention can be combined with each other to form a novel or preferred technical solution. The details would not be described one by oneherein due to the limitation of space.

### BRIEF DESCRIPTION OF THE DRAWINGS

In the figures of this application, "Untreated T" refers to untreated T cells, "Control" refers to T cells electroporated only with homologous templates and Cas9, "LV-19bbz" refers to CD19-CART cells prepared with lentivirus, "AAVS1-19bbz" refers to non-viral AAVS1 site-directed integrated CD19-CART cells, and "PD1-19bz" refers to non-viral PD1 site-directed integrated CD19-CART cells.
Figure 1 is a schematic diagram of the principle for the construction of non-viral site-directed integrated CAR-T cells. "TRAC-19bbz" indicates non-viral TRAC site-directed integrated CD19-CART cells, and "B2M-19bbz" indicates non-viral B2M site-directed integrated CD19-CART cells.
Figure 2 shows T cell viability after electroporation of plasmid or linear double-stranded DNA. Only plasmid or linear double-stranded DNA was electroporated in the control group, and templates, Cas9, and sgRNA were electroporated simultaneously in the sample group.
Figure 3 shows mTurquoise2 positive rate after electroporation using different templates. The control group was electroporated with HDR templates and Cas9 only, and the sample group was electroporated with linear double-stranded DNA templates, Cas9 and sgRNA. The protective bases were added on both sides in the "pb" group.
Figure 4 shows mTurquoise2 positive rate and cell viability after electroporation of templates with different lengths of homology arms. The control group was electroporated with the template containing 800 bp homology arms and Cas9 only, and the sample group was electroporated with the templates with different lengths of homology arms, Cas9 and sgRNA; panel A shows the positive rate of mTurquoise2, panel B shows the relative number of viable cells, and panel C shows the relative number of mTurquoise2-positive viable cells.
Figure 5 shows the screening of AAVS1 sgRNA. Panel A shows the knock-out rate of AAVS1 locusdetected by T7E1, and panel B shows the recombination rate of fluorescent protein mTurquioise2 sequence at AAVS1 locus. In the figure, "sgRNA-1" refers to AAVS1-sgRNA1 for this application, "sgRNA-2" refers to AAVS1-sgRNA2 for this application, "sgRNA-3" refers to AAVS1-sgRNA3 for this application, and "sgRNA-4" refers to AAVS1-sgRNA4 in this application.
Figure 6 shows the screening of PD1 sgRNA and the recombination rate of the fluorescent protein mTurquioise2 sequence at the PD1 site. In the figure, "sgRNA-1" indicates PD1-sgRNA1 for this application and "sgRNA-2" indicates PD1-sgRNA2 for this application.
Figure 7 shows the screening of TRAC sgRNA. Panel A shows the knock-out rate of TRAC site by detecting CD3 expression and panel B shows the recombination rate of the fluorescent protein mTurquioise2 sequence at the TRAC site. In the figure, "sgRNA-1" refers to TRAC-sgRNA1 for this application, "sgRNA-2" refers to TRAC-sgRNA2 for this application, and "sgRNA-3" refers to TRAC-sgRNA3 for this application.
Figure 8 shows the screening of B2M sgRNA. Panel A shows the knock-out rate at the B2M site detected by B2M expression and panel B shows the recombination rate of the fluorescent protein mTurquioise2 sequence at the B2M site. In the figure, "sgRNA-1" indicates B2M-sgRNA1 for this application and "sgRNA-2" indicates B2M-sgRNA2 for this application.
Figure 9 shows CAR positive rate and cell viability after electroporation of templates of different lengths of homology arms. The control group was electroporated with the templates containing 800 bp homology arms and Cas9 only, and the sample group was electroporated with the templates with different lengths of homology arms, Cas9 and sgRNA; panel A shows CAR positive rate, panel B shows relative number of viable cells, and panel C shows relative number of CAR-positive viable cells.
Figure 10 shows positive rate of AAVS1 site-directed integrated CD19-CART cells in CD3+, CD4+, and CD8+ T cells.
Figure 11 shows positive rate and knock-out rate of AAVS1 site-directed integrated CD19-CART cells. Panel A shows positive rate of AAVS1 site-directed integrated CD19-CART cells prepared from different human donor cells; panel B shows positive rate and knock-out rate of AAVS1 site-directed integrated CD19-CART cells prepared from five representative different human donor cells; panel C shows positive rate of AAVS1 site-directed integrated CD19-CART cells by flow cytometry. Only homologous templates and Cas9 were electroporated in the control group.
Figure 12 shows the DNA sequencing results of AAVS1 site-directed integrated CD19-CART cells. HA stands for homology arm.
Figure 13 shows the viability of AAVS1 site-directed integrated T-cells.
Figure 14 shows *in vitro* expansion of AAVS1 site-directed integrated T cells.
Figure 15 shows the *in vitro* expansion of AAVS1 site-directed integrated CD19-CART cells when co-cultured with target cells.
Figure 16 shows the *in vitro* expansion of AAVS1 site-directed integrated CD19-CART cells when co-cultured with target cells detected by labeling experiments. Only homologous templates and Cas9 were electroporated in the control group.
Figure 17 shows the surface marker expression in AAVS1 site-directed integrated CD19-CART cells. Only homologous templates and Cas9 were electroporated in the control group.
Figure 18 shows the subtype proportion of AAVS1 site-directed integrated CD19-CART cells. Only homologous templates and Cas9 were electroporated in the control group.
Figure 19 shows the secreted cytokines by AAVS1 site-directed integrated CD19-CART cells. Only homologous templates and Cas9 were electroporated in the control group.
Figure 20 shows the *in vitro* killing ability of AAVS1 site-directed integrated CD19-CART cells. Only homologous templates and Cas9 were electroporated in the control group.
Figure 21 shows the *in vivo* killing ability of AAVS1 site-directed integrated CD 19-CART cells. Tumor target cells are Raji cells.

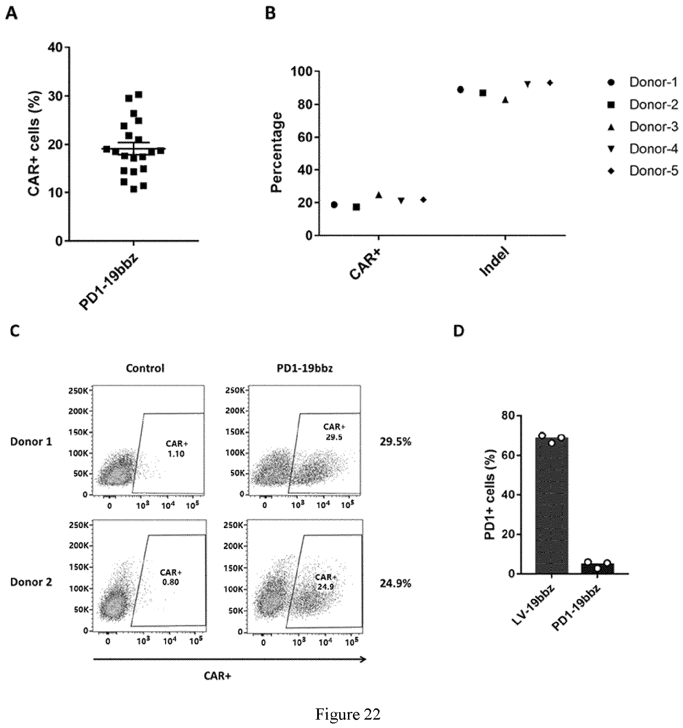

Figure 22 shows positive rate and knock-out rate of PD1 site-directed integrated CD19-CART cells. Panel A shows positive rate of PD1 site-directed integrated CD19-CART cells prepared from different human donor cells; panel B shows positive rate and knock-out rate of PD1 site-directed integrated CD19-CART cells prepared from five representative different human donor cells; panel C shows positive rate of PD1 site-directed integrated CD19-CART cells by flow cytometry; panel D shows comparison of PD1 expression between PD1 site-directed integrated CD19-CART cells and lentivirus-prepared CD19-CART cells. Only homologous templates and Cas9 were electroporated in the control group.
Figure 23 shows the DNA sequencing results of CD19-CART cells with PD1 site-directed integration. HA stands for homology arm.
Figure 24 shows the *in vitro* expansion of PD1 site-directed integrated CD19-CART cells when co-cultured with target cells.
Figure 25 shows the surface marker expression of PD1 site-directed integrated CD19-CART cells. Only homologous templates and Cas9 were electroporated in the control group.
Figure 26 shows the detection of secreted cytokines in PD1 site-directed integrated CD 19-CART cells. Only homologous templates and Cas9 were electroporated in the control group.
Figure 27 shows the *in vitro* killing ability of PD1 site-directed integrated CD19-CART cells. Only homologous templates and Cas9 were electroporated in the control group.
Figure 28 shows the *in vivo* killing ability of PD1 site-directed integrated CD19-CART cells. Tumor target cells were PD-L1-overexpressing Raji cells.

### DETAILED DESCRIPTION OF THE PREFERRED EMBODIMENTS

After extensive and intensive research, and through a large number of screening, the inventors accidentally screened gRNAs for highly efficient gene editing of target sites (such as: AAVS 1, PD 1, TRAC, B2M) for the first time. In addition, by comparing the first homology arm and the second homology arm of different lengths, the inventors further accidentally determined the conditions for highly efficient integration of the exogenous nucleic acid into the genome of T cells (such as highly efficient integration using the first homology arm and the second homology arm with specific lengths at genomic target sites). The present invention is completed on this basis.

### Terminology

### CRISPR/Cas9-mediated gene editing method

CRISPR/Cas9 is an adaptive immune defense formed by bacteria and archaea during long-term evolution and can be used to fight invading viruses and exogenous DNA. The CRISPR/Cas9 system provides immunity by integrating fragments of invading phage and plasmid DNA into CRISPR and using corresponding CRISPR RNAs (e.g., gRNAs) to guide the degradation of homologous sequences.

The working principle of this system is that crRNA (CRISPR-derived RNA) binds to tracrRNA (trans-activating RNA) through base pairing to form a tracrRNA/crRNA complex, which guides the nuclease Cas9 protein to cleave double-stranded DNA at the target site with the sequence paired with crRNA. While these two RNAs can be engineered to form gRNAs (single-guide RNAs) with guiding function by artificial design, they are sufficient to guide the site-directed cleavage of DNA by Cas9.

As an RNA-directed dsDNA-binding protein, Cas9 effector nuclease is the first known unifying factor and is able to co-localize RNA, DNA, and protein. Fusion of the protein to nuclease-free Cas9 (Cas9 nuclease-null) and expression of the appropriate gRNA can target any dsDNA sequence, while the end of the gRNA can be attached to the target DNA without affecting Cas9 binding. Therefore, Cas9 can bring any fusion protein and RNA at any dsDNA sequence. This technology is called the CRISPR/Cas9 gene editing system.

### Targeted integration mechanism

In the targeted integration strategy of the present invention, a linearized donor (i.e., DNA donor or exogenous nucleic acid) containing a homology arm with a specific length is provided, which is a transgenic donor template containing a homology arm with a certain length (e.g., 700-900 bp) obtained by PCR amplification or precise digestion; DNA donor or exogenous nucleic acid is electroporated into cells along with Cas9 mRNA and guide RNA. The targeted integration strategy provided by the present invention has higher integration efficiency than the existing gene targeting strategy.

In the present invention, a linearized dsDNA donor (i.e., DNA donor or exogenous nucleic acid) consisting of a transgene fragment and 700-900 bp homology arms on either of the two sides is a key factor for achieving highly efficient targeted integration by CRISPR-mediated genome editing. The targeted integration technique of the present invention has the highest recombination efficiency compared to MMEJ (microhomology arm-mediated end-joining) or HITI (homology-independent targeted integration) -mediated methods.

### Methods for gene editing of target sites in cellular genomes

The present invention provides a method for gene editing at a target site in a cellular genome, comprising:
(a) providing a cell to be gene-edited;
(b) introducing (i) an enzyme for gene editing, or a nucleic acid encoding the same, or a first expression vector expressing the enzyme for gene editing; and (ii) gRNA, or a second expression vector expressing the gRNA into the cell, and performing gene editing on a target site of the cellular genome, wherein the gRNA guides the enzyme for gene editing to perform site-directed cleavage on the target site;
wherein, the targeting sequence targeted by the gRNA comprises one or more of the sequences shown in SEQ ID NO: 1-9.

The methods of the present invention enables highly efficient gene knock-out or gene-targeted integration at target sites (such as AAVS1, PD1, TRAC, B2M, etc.).

### Reaction system

The present invention provides a reaction system for gene editing at a target site in a cellular genome comprising:
(a) a DNA donor, wherein the DNA donor is double-stranded DNA;
(b) an enzyme for gene editing or nucleic acid encoding the same, or a first expression vector expressing the enzyme for gene editing;
(c) a gRNA or a second expression vector expressing the gRNA;
wherein, the targeting sequence targeted by the gRNA comprises one or more of the sequences shown in SEQ ID NO: 1-9.

In a preferred embodiment, the DNA donor comprises a first homology arm and a second homology arm, wherein the first homology arm and the second homology arm are capable of initiating a homologous recombination of the DNA donor mediated by an immune cell at the target site in the immune cellular genome, and the sequence lengths of the first homology arm and the second homology arm are 200-2000 bp independently, preferably 400-1000 bp, more preferably 700-900 bp.

In the present invention, the sequence length of the DNA donor to be integrated can be 50 bp-5000 bp. The length of the target gene (or called target gene) in the DNA donor of the present invention may be 50 bp-3000 bp.

In the present invention, there is no special restriction on target sites, and one preferred target site includes AAVS1, PD1, TRAC, B2M.

In a preferred embodiment of the present invention, the ratio (D1/D2) of the sequence length of the first homology arm (D1) to the sequence length of the second homology arm (D2) is (0.8-1.2) : (0.5-1.5), preferably (0.9-1.1) : (0.7-1.3), more preferably 1:1.

In this reaction system, the sequence lengths of two homology arms are not required to be completely identical, and a certain length difference is allowed between them.

**The main advantages of the present invention include:**
1) The efficiency of gene editing (such as site-directed integration) of the method in the present invention is significantly higher than that of other existing gene editing technologies.
2) The introduction method is simple and can be introduced by electroporation and other means.
3) The present invention can improve the site-directed integration efficiency of exogenous nucleic acid in T cells.
4) The method of the present invention enables the preparation of site-directed integrated T cell products.
5) This method can successfully prepare non-viral site-directed integrated CAR-T cells at AAVS1, PD1, TRAC, B2M and other sites, which can avoid many drawbacks in virus preparation and improve the homogeneity and robustness of CAR-T products. In addition, it also provides technical support for the preparation of enhanced, inducible and other diverse CAR-T products.

The present invention is further elaborated in combination with specific embodiments. It should be understood that these embodiments are solely intended to describe the present invention and not to limit the scope of the present invention. Experimental methods that do not specify specific conditions in the following embodiments are generally performed according to conventional conditions such as those described in Sambrook et al., Molecular Cloning: Laboratory Manual (New York: Cold Spring Harbor Laboratory Press, 1989), or according to conditions described in the Microbiology: Laboratory Manual (James Cappuccino and Natalie Sherman, Pearson Education Press), or as suggested by the manufacturer. Human donor cells used in the experiments in the present invention were obtained from Shanghai SAILY Biological Technology Co., Ltd.

Materials and reagents used in the embodiments are commercially available unless otherwise specified.

### General Methods

### I. Electroporation of T cells

Electroporation of T cells is a technical method to achieve gene editing of T cells, and the procedure is based on Lonza P3 Primary Cell 4D-Nucleofector ^{®} X kit.

Instruments and materials:
① Lonza 4D-Nucleofector ^{™} System cell nucleofector
② Reagent kit is P3 Primary Cell 4D-Nucleofector^{™} X Kit, (Lonza, V4XP-3024)
③ T cells after CD3/CD28 magnetic bead stimulation for 2-4 days
④ Commercial spCas9 protein (5 µg/µL) (TrueCut^{™} Cas9 Proteinv2, Thermofisher)
⑤ Synthetic sgRNA (synthetic sgRNA was dissolved in TE buffer and diluted to a final concentration of 10 µg/µL)
⑥ Linearized double-stranded DNA containing homologous templates.

### Specific operation steps:

Applicable to 100 µL electroporation cuvette:
(1) In each electroporation cuvette, 82 µL Solution + 18 µL supplement were added. According to the total number of electroporation cuvettes, a mixture for electroporation reaction was prepared, mixed well and placed at room temperature.
(2) Cas9 protein and sgRNA9 were co-incubated at room temperature for 10 min to form RNP.
(3) Linearized double-stranded DNA homologous template (including fluorescent protein mTurquoise2 sequence containing homology arms at two sites of PD1, CD19-CART sequence containing homology arms at sites of AAVS1, PD1, TRAC, and B2M) was added to RNP and incubated at room temperature for 2 min.
   The CD19-CART sequence includes the extracellular domain targeting CD19, the transmembrane region of CD8α, and the intracellular signaling domains from CD3ζ and CD137.
(4) T cells in the activated state were collected and counted at 5×10⁶ for an electroporation reaction.
(5) The cells were resuspended and mixed thoroughly with the "RNP + homology template", and then added to the electroporation cuvette.
(6) The electroporation system was turned on, the electroporation cuvette was put into the slot hole, and the program EO115 for electroporation was selected.
(7) Cells were added to prewarmed cell culture medium and cultured in a cell incubator.

### II. Detection of recombination efficiency of exogenous sequence

1) 1 × 10⁶ cells were taken in a sterile 1.5 mL centrifuge tube, and the supernatant was discarded after centrifugation;
2) the cells were washed by adding the detection buffer (PBS containing 2% serum) to the cell pellet;
3) the cells were placed in a centrifuge at room temperature, and the supernatant was absorbed as much as possible after centrifugation;
4) the cells were co-incubated with the detection antibody or protein, and incubated on ice for 30 minutes;
5) the cells were washed twice with the detection buffer, and the supernatant was absorbed as much as possible after centrifugation;
6) the cells were resuspended with appropriate volume of the detection buffer for flow cytometry analysis;

### III. Viable Cell Number Testing

The Celltiter One Solution Cell Proliferation Assay kit from Promega was used to detect the number of viable cells according to the instructions.
1) MTS reagent was equilibrated to room temperature;
2) triplicate wells were set up, 100 µL of cell suspension was transferred to a 96-well plate, and 20 µL of MTS reagent was added;
3) the plate was incubated for 4 hours at 37°C in an incubator containing 5% CO₂;
4) the plate was detected by microplate reader at 490 nm, and the data were statistically analyzed.

For example, non-viral site-directed integrated CD19-CART cells (including T cells with CAR integrated at AAVS1 safe harbor, T cells with CD19-CAR integrated at PD1 immune checkpoint locus, T cells with CD19-CAR integrated at TRAC locus, and T cells with CD19-CAR integrated at B2M locus) can be constructed in one step using the method of the invention combined with CRISPR/Cas9 technology.
1. Target sequence information
   AAVS1-sgRNA1: CCGGAGAGGACCCAGACACG (SEQ ID NO: 7)
   AAVS1-sgRNA2: AGAGCTAGCACAGACTAGAG (SEQ ID NO: 3)
   AAVS1-sgRNA3: AAAGCAAGAGGATGGAGAGG (SEQ ID NO: 8)
   AAVS1-sgRNA4: GAGAGGACCCAGACACGGGG (SEQ ID NO: 9)
   PD1-sgRNA1: CGACTGGCCAGGGCGCCTGT (SEQ ID NO: 1)
   PD1-sgRNA2: GGGCGGTGCTACAACTGGGC (SEQ ID NO: 2)
   TRAC-sgRNA1: ACAAAACTGTGCTAGACATG (SEQ ID NO: 4)
   TRAC-sgRNA2: AGAGCAACAGTGCTGTGGCC (SEQ ID NO: 5)
   TRAC-sgRNA3: AGAGTCTCTCAGCTGGTACA (SEQ ID NO: 10)
   B2M-sgRNA1: ACTCACGCTGGATAGCCTCC (SEQ ID NO: 11)
   B2M-sgRNA2: GAGTAGCGCGAGCACAGCTA (SEQ ID NO: 6)
2. sgRNA was synthesized by the company and dissolved in TE buffer, and diluted to a final concentration of 10 µg/µL.
3. Preparation of non-viral site-directed integrated CD19-CART

### Instruments and materials:

① Lonza 4D-Nucleofector ^{™} System cell nucleofector
② Reagent kit is P3 Primary Cell 4D-Nucleofector ^{™} X Kit, (Lonza, V4XP-3024)
③ T cells after CD3/CD28 magnetic bead stimulation for 2-4 days
(4) Commercial spCas9 protein (5 µg/µL) (TrueCut^{™} Cas9 Proteinv2, Thermofisher)
⑤ SgRNA synthesized by the company
⑥ Linearized double-stranded DNA containing homologous templates.

### Specific operation steps:

Applicable to 100 µL electroporation cuvette:
(1) In each electroporation cuvette, 82 µL Solution + 18 µL supplement were added. According to the total number of electroporation cuvettes, a mixture for electroporation reaction was prepared, mixed well and placed at room temperature.
(2) Cas9 protein was co-incubated with AAVS1-sgRNA, PD1-sgRNA1, PD1-sgRNA2, TRAC-sgRNA, and B2M-sgRNA, respectively, at room temperature for 10 min to form RNP.
(3) Linearized double-stranded DNA containing homology arms at each site was added to RNP and incubated at room temperature for 2 min.
(4) T cells in the activated state were collected and counted at 5×10⁶ for an electroporation reaction.
(5) The cells were resuspended and mixed thoroughly with the "RNP + homology template" and added to the electroporation cuvette.
(6) The electroporation system was turned on, the electroporation cuvettes were put into the slot hole, and the program EO115 for electroporation was selected.
(7) Cells were added to prewarmed cell culture medium and cultured in a cell incubator.

### 4. Evaluation of AAVS1 site-directed integrated CD19-CART cells

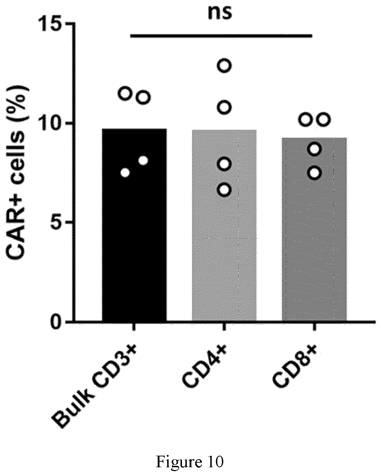

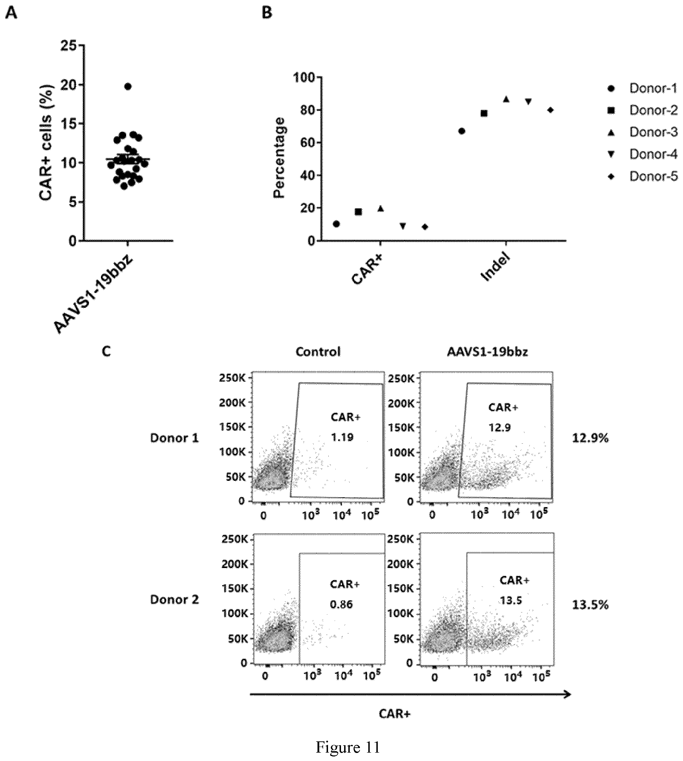

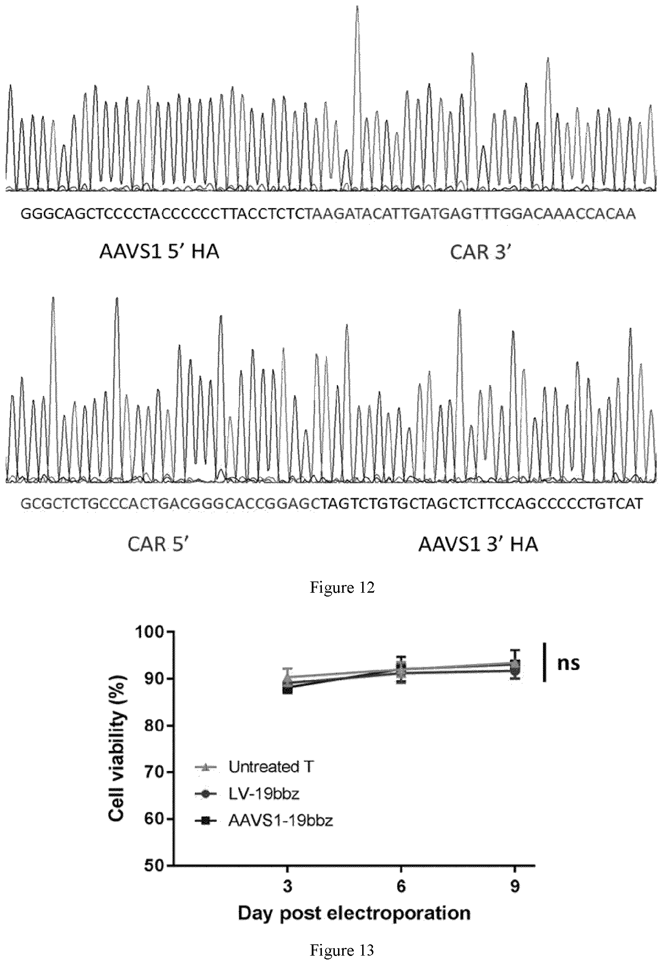

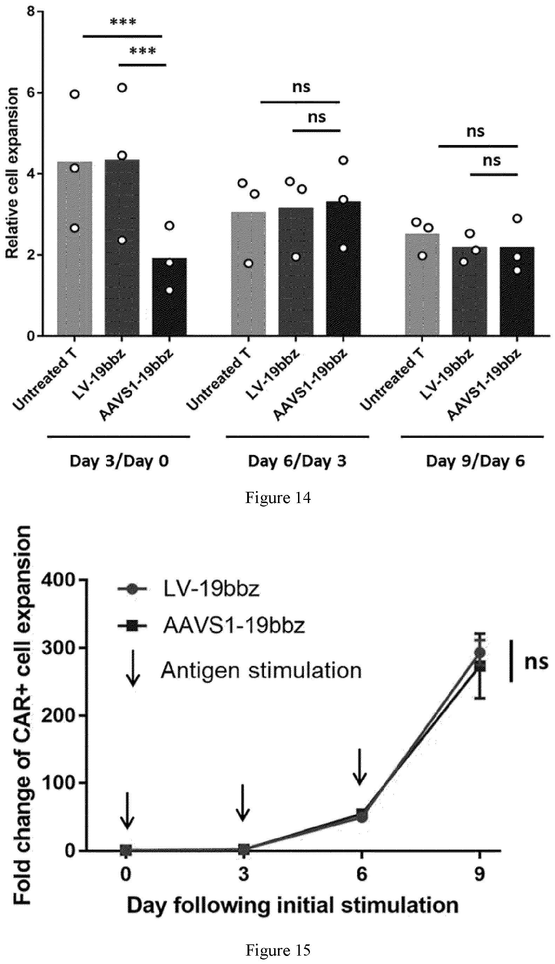

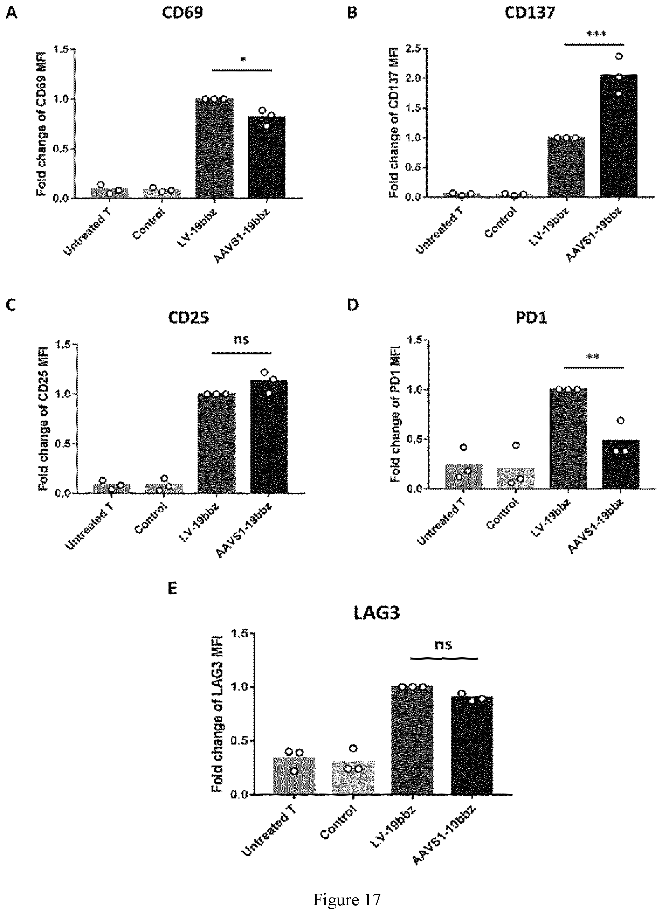

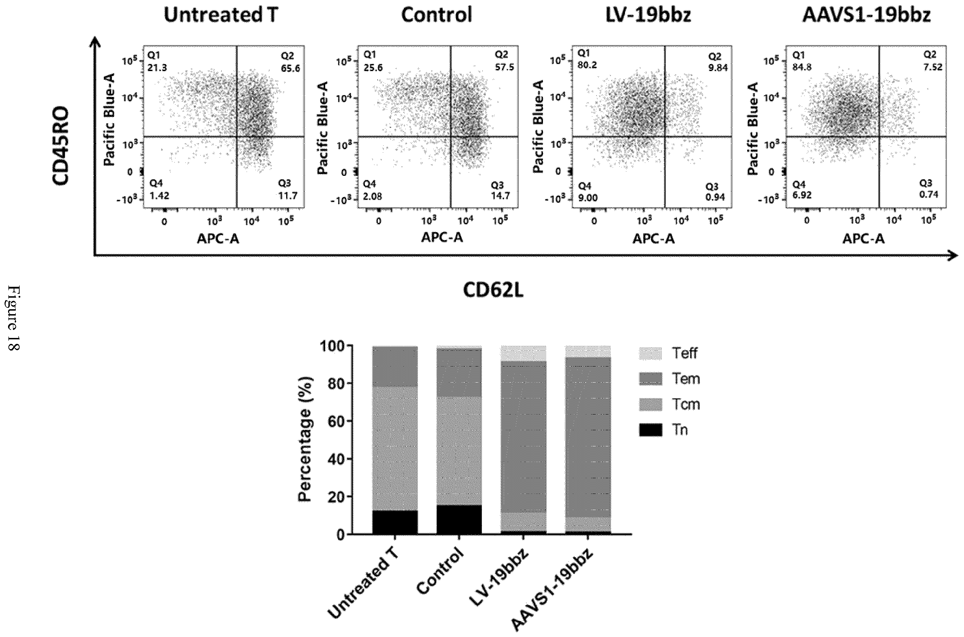

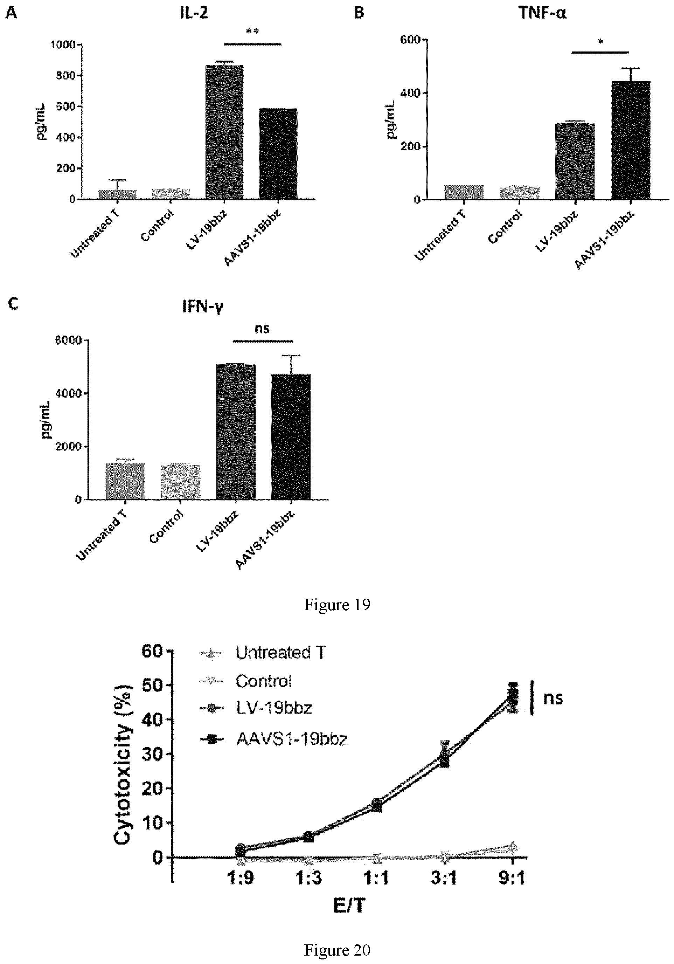

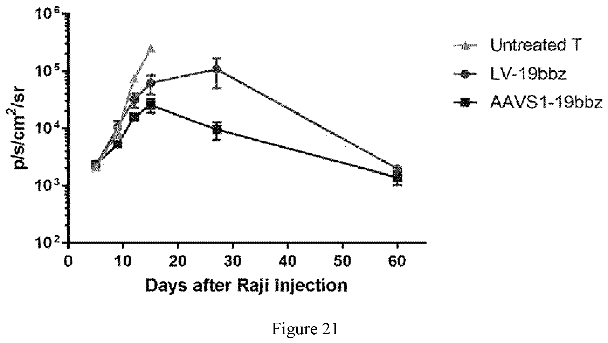

(1) By using the present invention, AAVS1 site-directed integrated CAR-T cells were constructed without the usage of viruses. The CAR positive rate was approximately 10%-20% (Figure 11), and the knock-out rate was approximately 65%-90% (Figure 11), and the site-directed integration of CAR elements could be achieved in CD3+, CD4+, and CD8+ T cells by this method (Figure 10).
(2) The AAVS1 site-directed integrated CD19-CART cells constructed using the present invention have a high viability (Figure 13). Although the electroporation step itself caused some cell death, it did not affect the efficient expansion of AAVS1 site-directed integrated CD 19-CART cells (Figures 14-16).
(3) Although there are some differences, in general, AAVS1 site-directed integrated CD19-CART cells presented similar characteristics in cell surface marker expression, cell subtype changes, and cytokine secretion compared with CD19-CART cells prepared by lentiviruses (Figures 17-19).
(4) Similar to CD19-CART cells prepared by lentivirus, AAVS1 site-directed integrated CD19-CART cells constructed by this invention can effectively kill tumor cells both *in vitro* and *in vivo* (Figures 20 and 21).

### 5. Evaluation of PD 1 site-directed integrated CD 19-CART cells

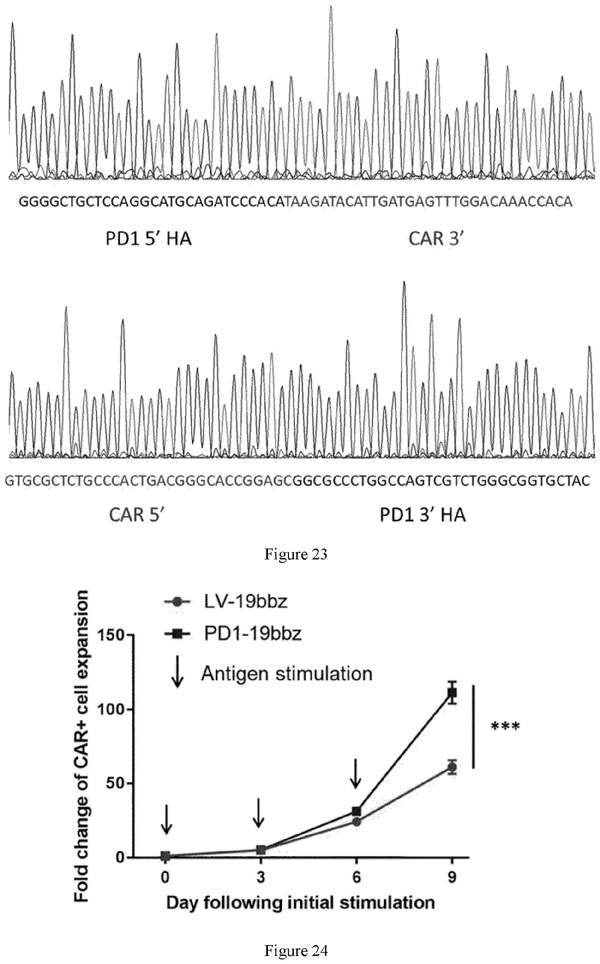

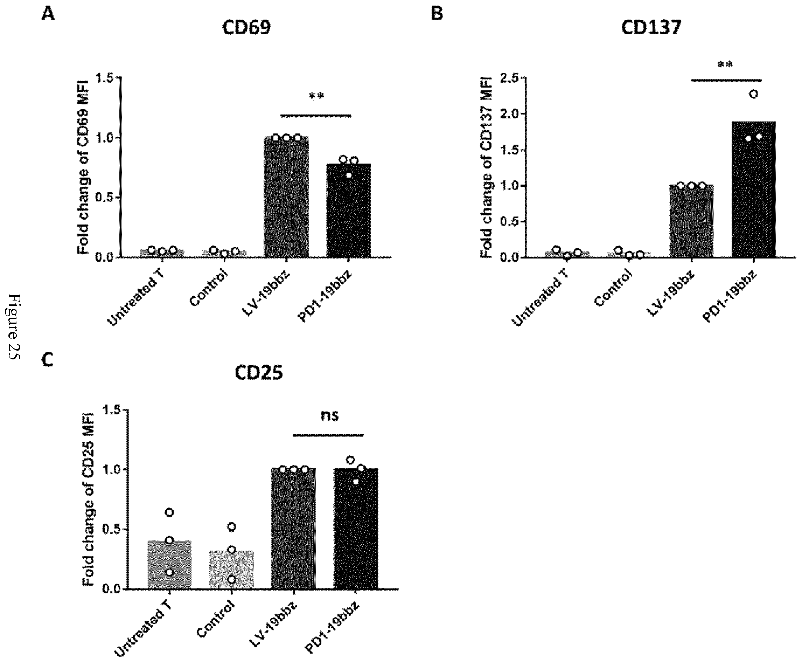

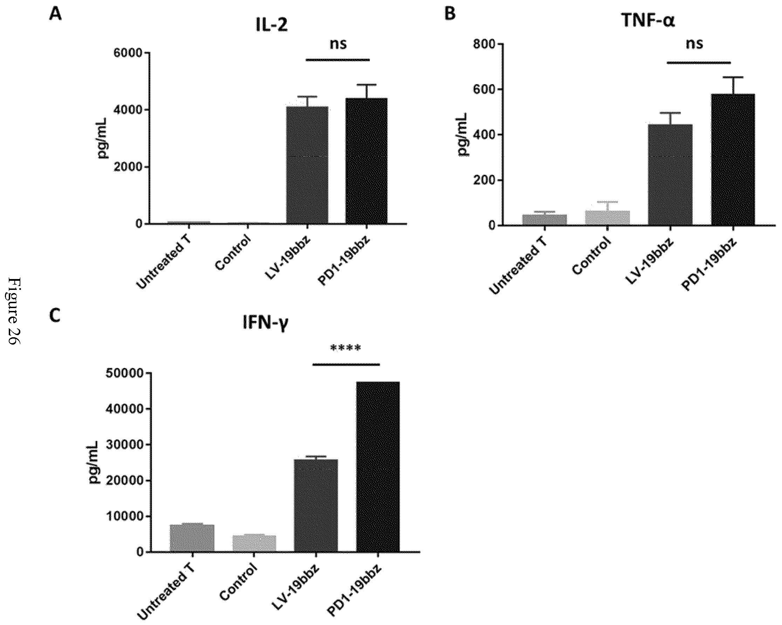

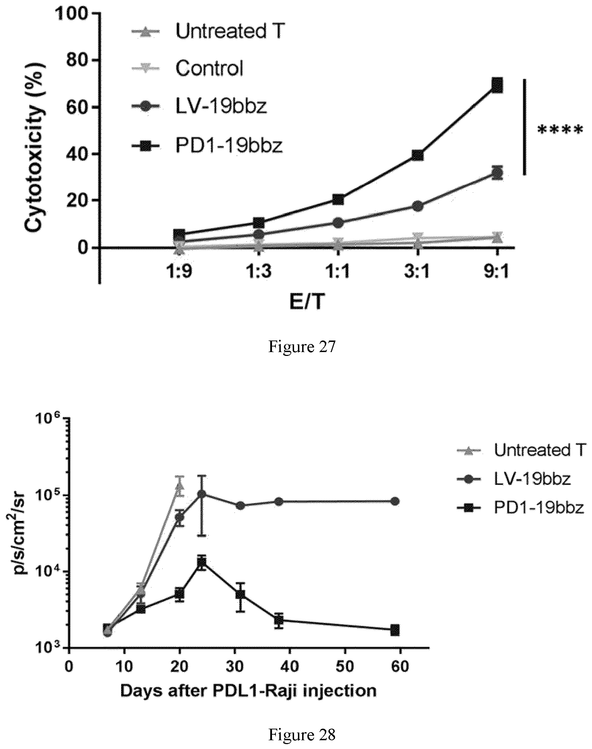

(1) By using the present invention, PD1 site-directed integrated CAR-T cells were constructed without the usage of viruses. The CAR positive rate was approximately 10%-30% (Figure 22) and the knock-out rate was approximately 80%-95% (Figure 22).
(2) The PD1 site-directed integrated CD19-CART cells constructed using the present invention have a stronger ability of cell expansion than CD19-CART cells prepared by lentivirus (Figure 24).
(3) Similar to CD19-CART cells prepared by lentivirus, the expression of activation surface markers was upregulated in PD1 site-directed integrated CD19-CART cells in response to tumor cells, with a more pronounced increase in CD137 expression (Figure 25).
(4) Similar to CD19-CART cells prepared by lentivirus, PD1 site-directed integrated CD19-CART cells could secrete cytokines in response to tumor cells, with a more pronounced increase in IFN-y secretion (Figure 26).
(5) Compared with CD 19-CART cells prepared by lentivirus, PD1 site-directed integrated CD19-CART cells constructed by this invention had stronger anti-tumor ability *in vitro* and *in vivo* (Figures 27 and 28).

In addition, it should be mentioned that this application uses linear double-stranded DNA (DNA donor) as an example to perform experiments as shown below.

Among them, the plasmid sequence is described as follows:
(1) The mTurquoise2 sequence of a plasmid donor with 800 bp homology arms which is site-directed integrated at PD1 site 1 was used as an example to illustrate the situation of vector and DNA donor:
   the vector was a circular structure, and the vector backbone was obtained from plasmid pmaxGFP^{™} and purchased from Lonza Company.
   The sequence of DNA donor is identical to the "PD1 site 1 with site-directed integration of the mTurquoise2 sequence with 800 bp homology arms (HDR)" in the linear double-stranded DNA sequence, i.e. as shown in SEQ ID NO: 12.
(2) The mTurquoise2 sequence with 800 bp homology arms site-directed integrated at PD1 site 2 was used as an example to illustrate the situation of vector and DNA donor:
   the vector was a circular structure, and the vector backbone was obtained from plasmid pmaxGFP^{™} and purchased from Lonza Company.

The sequence of DNA donor is identical to the "PD1 site 2 with site-directed integration of the mTurquoise2 sequence with 800 bp homology arms (HDR)" in the linear double-stranded DNA sequence, i.e. as shown in SEQ ID NO: 13.

Linear double stranded DNA (DNA donor) is shown in any one of SEQ ID NO: 12-38.

Among them, the mTurquoise2 sequence with 800 bp homology arms (HDR) site-directed integrated at PD1 site 1 is shown in SEQ ID NO: 12, the mTurquoise2 sequence with 800 bp homology arms (HDR) site-directed integrated at PD1 site 2 is shown in SEQ ID NO: 13, the mTurquoise2 sequence with 200 bp homology arms (HDR) site-directed integrated at PD1 site 1 is shown in SEQ ID NO: 14, the mTurquoise2 sequence with 400 bp homology arms (HDR) site-directed integrated at PD1 site 1 is shown in SEQ ID NO: 15, the mTurquoise2 sequence with 1600 bp homology arms (HDR) site-directed integrated at PD1 site 1 is shown in SEQ ID NO: 16, the mTurquoise2 sequence with 200 bp homology arms (HDR) site-directed integrated at PD1 site 2 is shown in SEQ ID NO: 17, the mTurquoise2 sequence with 400 bp homology arms (HDR) site-directed integrated at PD1 site 2 is shown in SEQ ID NO: 18, the mTurquoise2 sequence with 1600 bp homology arms (HDR) site-directed integrated at PD1 site 2 is shown in SEQ ID NO: 19, the mTurquoise2 sequence with 800 bp homology arms (HDR) site-directed integrated at AAVS1 site 1/4 is shown in SEQ ID NO: 20, the mTurquoise2 sequence with 800 bp homology arms (HDR) site-directed integrated at AAVS1 site 2 is shown in SEQ ID NO: 21, the mTurquoise2 sequence with 800 bp homology arms (HDR) site-directed integrated at AAVS1 site 3 is shown in SEQ ID NO: 22, the mTurquoise2 sequence with 800 bp homology arms (HDR) site-directed integrated at TRAC site 1 is shown in SEQ ID NO: 23, the mTurquoise2 sequence with 800 bp homology arms (HDR) site-directed integrated at TRAC site 2 is shown in SEQ ID NO: 24, the mTurquoise2 sequence with 800 bp homology arms (HDR) site-directed integrated at TRAC site 3 is shown in SEQ ID NO: 25, the mTurquoise2 sequence with 800 bp homology arms (HDR) site-directed integrated at B2M site 2 is shown in SEQ ID NO: 26, the CD19-CAR sequence with 200 bp homology arms (HDR) site-directed integrated at AAVS1 site 2 is shown in SEQ ID NO: 27, the CD19-CAR sequence with 400 bp homology arms (HDR) site-directed integrated at AAVS1 site 2 is shown in SEQ ID NO: 28, the CD19-CAR sequence with 800 bp homology arms (HDR) site-directed integrated at AAVS1 site 2 is shown in SEQ ID NO: 29, the CD19-CAR sequence with 200 bp homology arms (HDR) site-directed integrated at PD1 site 1 is shown in SEQ ID NO: 30, and the CD19-CAR sequence with 400 bp homology arms (HDR) site-directed integrated at PD1 site 1 is shown in SEQ ID NO: 31, the CD19-CAR sequence with 800 bp homology arms (HDR) site-directed integrated at PD1 site 1 is shown in SEQ ID NO: 32. The CD19-CAR sequence with 200 bp homology arms (HDR) site-directed integrated at TRAC site 2 is shown in SEQ ID NO: 33, the CD19-CAR sequence with 400 bp homology arms (HDR) site-directed integrated at TRAC site 2 is shown in SEQ ID NO: 34, the CD19-CAR sequence with 800 bp homology arms (HDR) site-directed integrated at TRAC site 2 is shown in SEQ ID NO: 35, the CD19-CAR sequence with 200 bp homology arms (HDR) site-directed integrated at B2M site 2 is shown in SEQ ID NO: 36, the CD19-CAR sequence with 400 bp homology arms (HDR) site-directed integrated at B2M site 2 is shown in SEQ ID NO: 37, the CD19-CAR sequence with 800 bp homology arms (HDR) site-directed integrated at B2M site 2 is shown in SEQ ID NO: 38.

Sequences of linear double-stranded DNA (DNA donor) as a comparative example are shown in any one of SEQ ID NO: 39-44.

Among them, the mTurquoise2 sequence with 800 bp homology arms (HITI-pb or writing"HITI (pb)") site-directed integrated at PD1 site 1 is shown in SEQ ID NO: 39, the mTurquoise2 sequence with 800 bp homology arms (HITI) site-directed integrated at PD1 site 1 is shown in SEQ ID NO: 40, the mTurquoise2 sequence with 800 bp homology arms (MMEJ) site-directed integrated at PD1 site 1 is shown in SEQ ID NO: 41, the mTurquoise2 sequence with 800 bp homology arms (HITI-pb or writing"HITI (pb)") site-directed integrated at PD1 site 2 is shown in SEQ ID NO: 42, the mTurquoise2 sequence with 800 bp homology arms (HITI) site-directed integrated at PD1 site 2 is shown in SEQ ID NO: 43, the mTurquoise2 sequence with 800 bp homology arms (MMEJ) site-directed integrated at PD1 site 2 is shown in SEQ ID NO: 44.

In the figures of this application, "Untreated T" refers to untreated T cells, "Control" refers to T cells electroporated only with homologous templates and Cas9, "LV-19bbz" refers to CD19-CART cells prepared by lentivirus, "AAVS1-19bbz" refers to non-viral AAVS1 site-directed integrated CD19-CART cells, and "PD1-19bz" refers to non-viral PD1 site-directed integrated CD19-CART cells.

### Example 1: double-stranded DNA as homologous template is superior to plasmid; when the homology arm of homologous template is set at 800 bp length, it has the best recombination efficiency and can obtain the largest amount of positive cells, which is the best condition

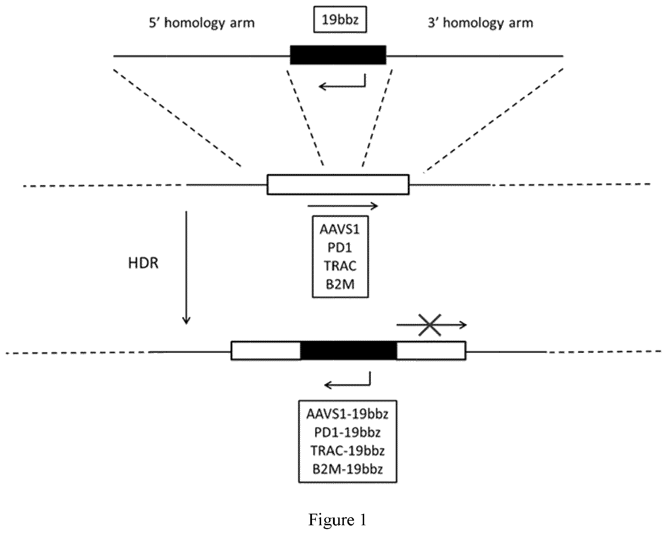

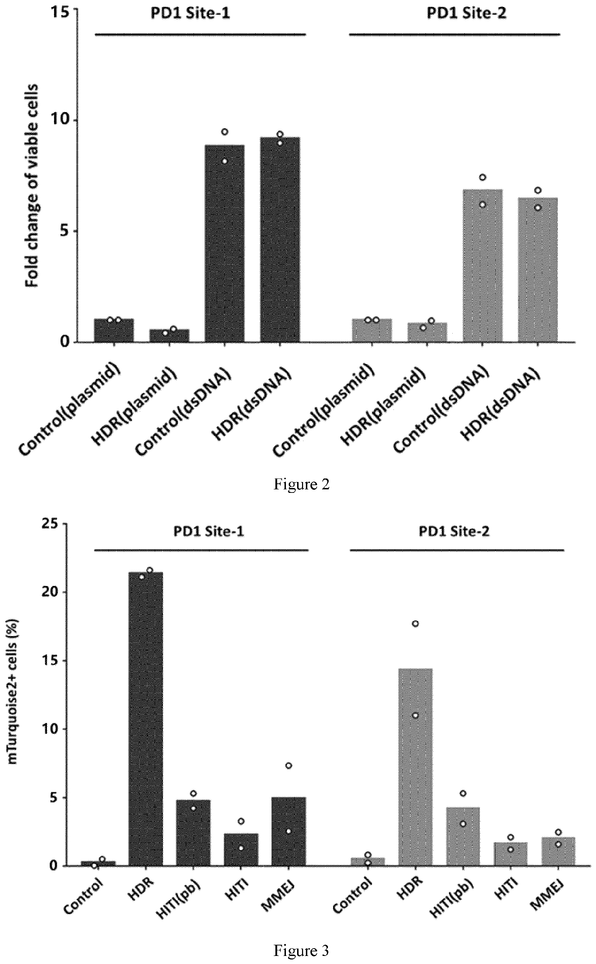

The effect of DNA templates in the form of plasmid and linear double-stranded DNA on T cells was first compared. The results showed that the viability of cells was significantly higher than that of using plasmids when templates were linear double-stranded DNA (Figure 2). Secondly, the effects of different template designs on recombination efficiency were compared, in which HDR was a 800bp homology arm template, MMEJ was a 20 bp microhomology arm template, HITI was a template having two-sided sgRNA target sequences without homology arms, HITI (pb) was a template on the basis of HITI with a 50-bp protecting base at both ends, and the donor sequences of HITI; HITI (pb), and MMEJ for PD1 site 1 were shown in SEQ ID NO: 39-41, respectively, and the donor sequences of HITI, HITI (pb), and MMEJ for PD1 site 2 were shown in SEQ ID NO: 42-44, respectively. The results showed that HDR templates had the highest recombination efficiency (Figure 3). Based on the above results, it demonstrated that it was the best method to construct non-viral site-directed integrated CAR-T cells using linear double-stranded DNA containing the HDR homology arm as a template (Figure 1).

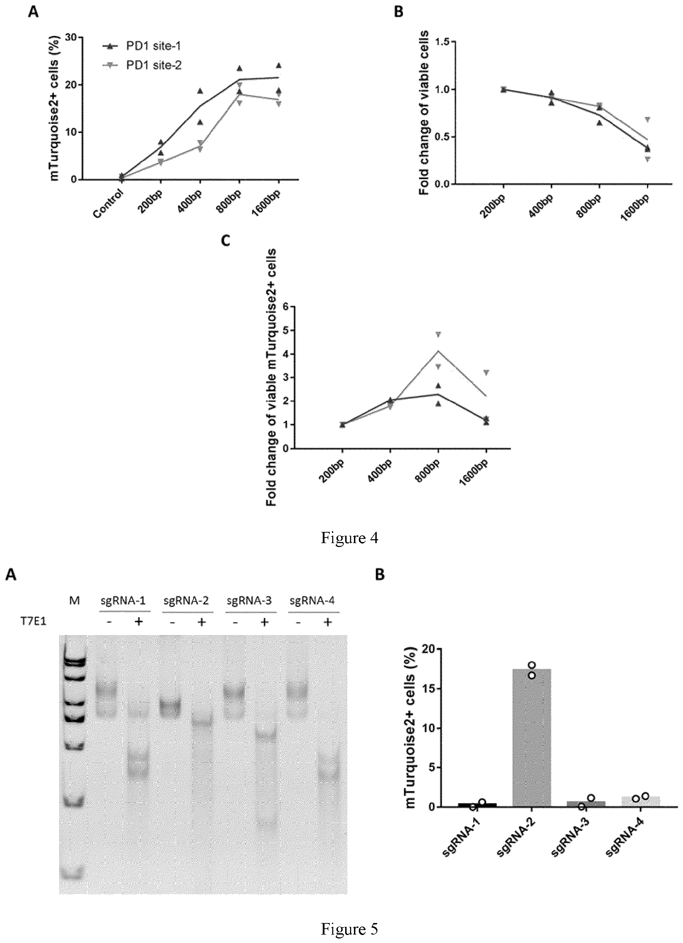

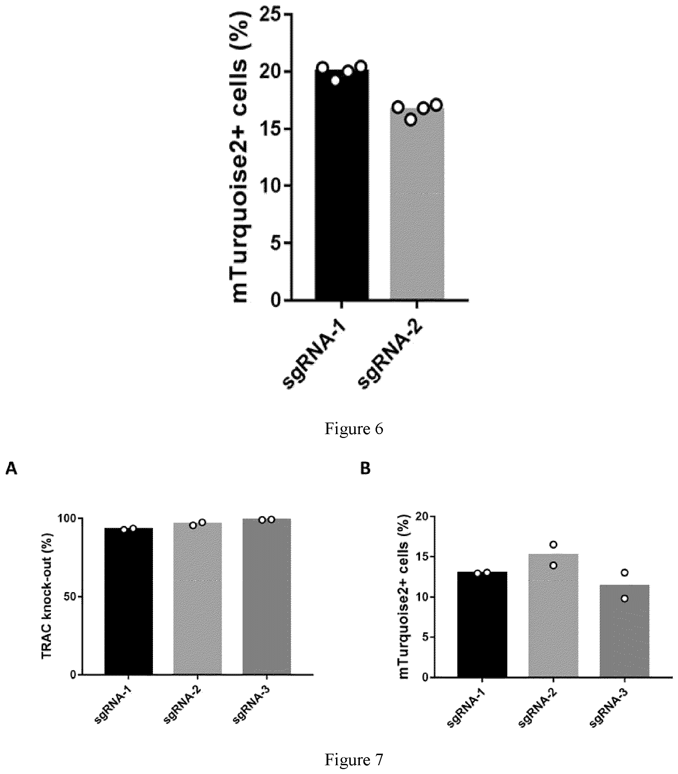

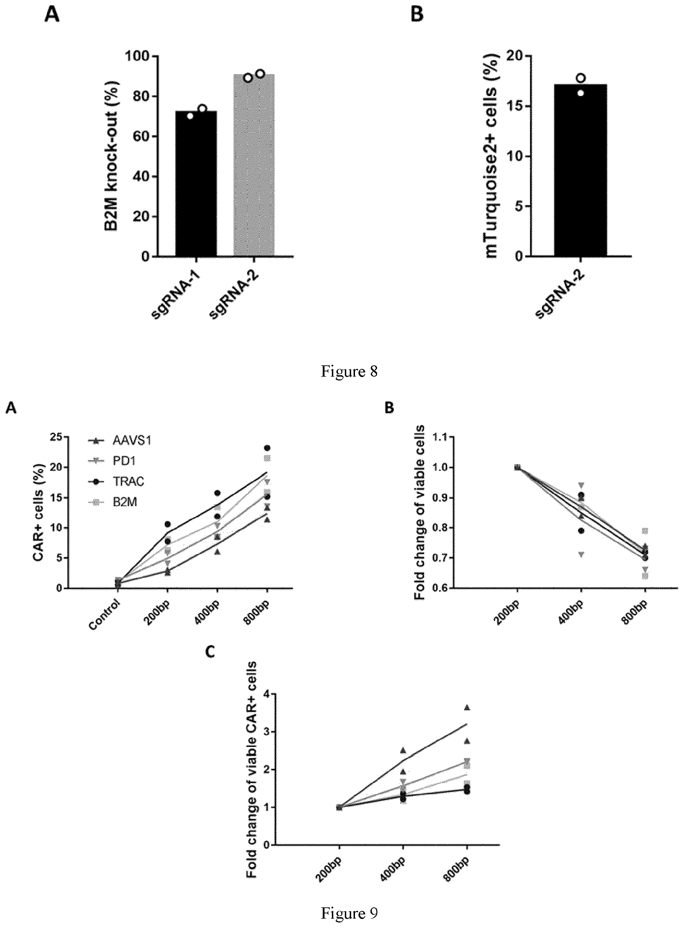

Following this, the effect of homology arm length was compared. Recombination efficiency tests were first carried out at two PD1 sites using the fluorescent protein mTurquoise2 sequence as the exogenous sequence (Figure 4). The results showed good integration effect, when the length of homology arm was 200 bp, 400 bp, 800 bp or 1600 bp, the integration rate of mTurquoise2 gradually increased with the increase of homology arm length, but the 1600 bp condition did not show a significant increase in recombination efficiency compared with the 800 bp condition. However, there was a significant negative correlation between cell viability and homology arm length. Combining recombination efficiency and cell viability, we analyzed the number of viable cells positive for mTurquoise2. The results showed that the largest amount of positive cells could be obtained when the homology arm length was 800 bp. On this basis, the efficient integration at PD1 sites were verified again and sites at AAVS1, TRAC, and B2M for efficient integration were screened. PD1-sgRNA1, AAVS1-sgRNA2, TRAC-sgRNA2, B2M-sgRNA2 were finally identified as preferred sequences by comparison of knock-out rate and recombination efficiency of mTurquoise2 (Figures 5-8). Finally, AAVS1, PD1, TRAC and B2M site-directed integrated CD19-CART cells were constructed using CAR elements as exogenous sequences and the effects of different lengths of homology arms were compared. Consistent with the mTurquoise2 results, the highest number of positive cells could be obtained when the homology arm was 800 bp (Figure 9). Therefore, we identify the HDR template containing 800 bp homology arms as the optimal condition for the construction of site-directed integrated T cells.

In previous experiments, we mainly explored and optimized the site-directed integration conditions for CD3+ T cells. On this basis, we attempted to edit CD4+ and CD8+ T cells using the same approach. The results showed that our method had similar site-directed integration rates in CD3+, CD4+, and CD8+ T cells (Figure 10).

### Example 2: Using the method of the present invention to construct non-viral AAVS1 site-directed integrated CD19-CART cells and testing their function

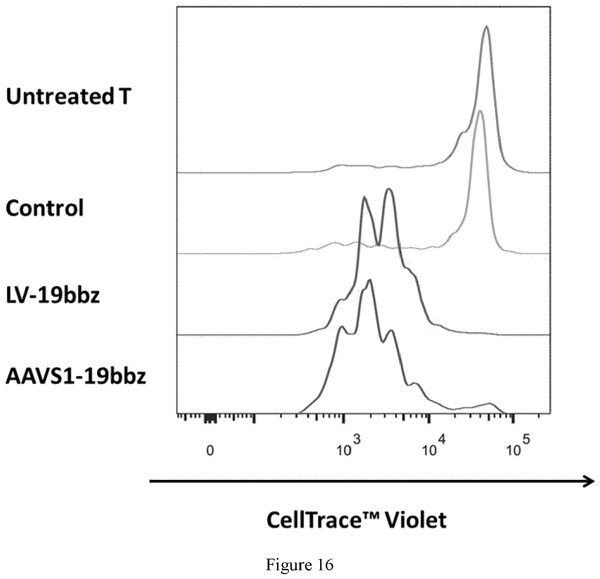

Using this method of the present invention, we successfully constructed AAVS1 site-directed integrated CD19-CART cells in different donor cells and verified them by sequencing, with an overall CAR positive rate of approximately 10%-20% and a knock-out rate of approximately 65%-90%, reflecting the robustness of cell preparation (Figures 11 and 12). On this basis, we examined the biological function of AAVS1 site-directed integrated CD19-CART cells. The experimental results showed that AAVS1 site-directed integrated CD19-CART cells had a high viability (Figure 13). Although the electroporation step itself caused some cell death, the ability of T cells to expand *in vitro* was not significantly compromised (Figure 14). AAVS1 site-directed integrated CD19-CART cells also expanded well after co-culture with tumor target cells (Figures 15 and 16). Afterwards, cell surface marker expression, cell subtype changes, and cytokine secretion were examined. In general, AAVS1 site-directed integrated CD19-CART cells responded significantly to tumor cells, similar to lentivirus-produced CD19-CART cells (Figures 17-19), although there are still some differences between them. Finally, tumor killing ability of AAVS1 site-directed integrated CD19-CART cells was assessed. In *vitro* and *in vivo* results demonstrated that AAVS1 site-directed integrated CD 19-CART cells had a good anti-tumor function as lentivirus-produced CD19-CART cells (Figures 20 and 21).

### Example 3: Construction of non-viral PD1 site-directed integrated CD19-CART cells using the method of the invention and testing their function

Using this method, we successfully constructed PD1 site-directed integrated CD19-CART cells in different donor cells and verified them by sequencing, with an overall CAR positive rate of approximately 10%-30% and a knock-out rate of approximately 80%-95%, reflecting the robustness of cell preparation (Figures 22 and 23). On this basis, we examined the biological function of PD1 site-directed integrated CD19-CART cells. The experimental results showed that PD1 site-directed integrated CD19-CART cells had a stronger ability of cell expansion than lentivirus prepared CAR-T cells after co-culture with tumor target cells (Figure 24). Similar to CD19-CART cells prepared by lentivirus, the expression of activation surface markers and secretion of cytokines in PD1 site-directed integrated CD19-CART cells were upregulated in response to tumor cell stimulation, with a more pronounced increase in CD137 expression (Figure 25) and a more pronounced increase in IFN-y secretion (Figure 26). Finally, *in vitro* and *in vivo* experiments demonstrated that PD1 site-directed integrated CD19-CART cells constructed using the present invention had a stronger anti-tumor ability compared to CD19-CART cells prepared by lentiviruses (Figures 27 and 28).

In summary, AAVS1, PD1, TRAC, and B2M site-directed integrated CAR-T cells can be successfully constructed by the CRISPR/Cas9 gene editing tool using the present invention method combined with site selection. The present invention proves that the site-directed integrated CAR-T cells prepared by the method of the present invention have a higher positive rate and can function effectively when compared with the prior art. Compared with traditional lentiviral preparation methods, this technique can reduce the high cost caused by the use of viruses in the preparation of CAR-T, reduce the potential safety risks caused by random insertion when using viruses, and also improve the homogeneity of CAR-T products. In addition, this method can also be applied to construct diverse CAR-T cells and enhance the anti-tumor ability of CAR-T cells. This example proves the importance and value of the method of improving the site-directed integration of exogenous sequences in T cells protected by the present invention, but is not limited to the preparation of AAVS1, PD1, TRAC and B2M site-directed integrated CAR-T cells, which can be extended to the site-directed integration of exogenous sequences at other sites and the development of other T cell immunotherapies.

All references mentioned in this invention are cited in this application as references, just as each article is cited separately as references. It is also understood that, having read the foregoing lectures on the present invention, a skilled person in the art may make various modifications or modifications to the present invention in such equivalent form as would fall within the scope of the claims attached to this application.

## Claims

1. A method for performing gene editing on a target site of a cellular genome comprising:
(a) providing a cell to be gene-edited;
(b) introducing (i) an enzyme for gene editing, or a nucleic acid encoding the same, or a first expression vector expressing the enzyme for gene editing; and (ii) gRNA, or a second expression vector expressing the gRNA into the cell, and performing gene editing on a target site of the cellular genome, wherein the gRNA guides the enzyme for gene editing to perform site-directed cleavage on the target site;
wherein, the targeting sequence targeted by the gRNA comprises one or more of the sequences shown in SEQ ID NO: 1-9.

2. The method of claim 1, wherein the gene editing comprises gene knock-out and gene-targeted integration.

3. The method of claim 1, wherein the enzyme for gene editing is selected from the group consisting of CRISPR related protein (Cas) polypeptide, TALEN enzyme, ZFN enzyme, or combinations thereof.

4. The method of claim 1, wherein the target site is selected from the group consisting of AAVS1, PD1, TRAC, B2M, or combinations thereof.

5. The method of claim 1, wherein the gene editing comprises a site-directed knock-in of a DNA donor.

6. A gRNA for gene editing on a target site of a cellular genome, wherein the targeting sequence targeted by the gRNA comprises one or more of the sequences shown in SEQ ID NO: 1-9.

7. A reaction system for gene editing on a target site of a cellular genome comprising:
(a) a DNA donor, wherein the DNA donor is double-stranded DNA;
(b) an enzyme for gene editing or nucleic acid encoding the same, or a first expression vector expressing the enzyme for gene editing;
(c) a gRNA or a second expression vector expressing the gRNA;
wherein, the targeting sequence targeted by the gRNA comprises one or more of the sequences shown in SEQ ID NO: 1-9.

8. A kit for gene editing comprising:
i) a first container and a DNA donor contained therein, wherein the DNA donor is double-stranded DNA;
ii) a second container and an enzyme for gene editing, or a nucleic acid encoding thereof or a first expression vector expressing the enzyme for gene editing contained therein; and
iii) a third container and a gRNA or a second expression vector expressing the gRNA contained therein, and the targeting sequence targeted by the gRNA comprises one or more of the sequences shown in SEQ ID NO: 1-9.

9. A gene-edited cell prepared by the method of claim 1.

10. A use of the cell of claim 9 in the preparation of a product for tumor immunotherapy or cancer immunotherapy.
